# EUROPEAN PATENT SPECIFICATION

(11) **EP 2 729 559 B1**
(45) Date of publication and mention of the grant of the patent: **22.08.2018**
(21) Application number: 12732667.6
(22) Date of filing: 06.07.2012
(51) Int. Cl.: C12N 1/00, C12N 1/04, C12N 1/14, C12N 1/20

(54) **METHOD FOR REDUCING THE VISCOSITY OF A MICROORGANISM-CONTAINING SUSPENSION OR CONCENTRATE**
VERFAHREN ZUR REDUZIERUNG DER VISKOSITÄT EINER MIKROORGANISMUSHALTIGEN SUSPENSION ODER EINES KONZENTRATS
PROCÉDÉ DE RÉDUCTION DE LA VISCOSITÉ D'UNE SUSPENSION OU UN CONCENTRÉ CONTENANT DES MICRO-ORGANISMES

(30) Priority: 06.07.2011 EP 11305868
(43) Date of publication of application: 14.05.2014
(73) Proprietor: DuPont Nutrition Biosciences ApS, 1411 Copenhagen K (DK)
(72) Inventor: CADENEL, Michel, 37250 Montbazon (FR); CHAIGNEAU, Patrick, 86100 Chatellerault (FR); D'ANGLOZ, Guy, 86220 Les Ormes (FR); HENRI, Erwan, 86220 Les Ormes (FR); ROCHER, Jean Pierre, 38360 Sassenage (FR)
(74) Representative: DuPont EMEA
(86) International application number: PCT/EP2012/063241
(87) International publication number: WO 2013/004815

(56) References cited:
- EP-A1- 0 259 739
- EP-A1- 1 441 027
- EP-A2- 0 145 197
- WO-A1-2005/060937
- WO-A1-2010/028489
- US-A- 4 339 464
- US-A1- 2006 135 406
- DAVISKAS EVANGELIA ET AL: "Hyperosmolar agents and clearance of mucus in the diseased airway.", JOURNAL OF AEROSOL MEDICINE : THE OFFICIAL JOURNAL OF THE INTERNATIONAL SOCIETY FOR AEROSOLS IN MEDICINE SPRING 2006 LNKD- PUBMED:16551221, vol. 19, no. 1, 21 March 2006 (2006-03-21) , pages 100-109, XP002663327, ISSN: 0894-2684

## Description

### Field of the invention

The present invention provides a method for reducing the viscosity of a microorganism-containing suspension or concentrate and a method for obtaining a microorganism concentrate.

### Background of the invention

Before inoculation into products, microorganisms are cultured in order to provide a suspension containing large amounts of microorganisms.

The production of microorganisms, such as ferments used in the agronomics industry, is performed within a growth medium incorporating nutrient substrate diluted in water. This growth medium is initially seeded with microorganisms, which thus develop within a quasi-closed environment, except for the supply of neutralizing agent (so-called "batch" culture). This "batch" culture fermentation applied on an industrial scale is a simple way to harvest a concentrated biomass using centrifugation techniques, such as ultracentrifugation, or filtration techniques such as microfiltration or ultrafiltration but in some situations, the high concentration of microorganisms is difficult to obtain without suffering from a major loss of biomass in the installation (pipelines of the apparatus, fermentor, harvesting equipments...).

Other kinds of culture fermentation processes exist, such as continuous fermentation. It consists in a metered extraction from the growth medium simultaneously with a metered in feed of nutrient substrate, neutralizer and water. This procedure renders it possible to increase the production of microorganisms for a bioreactor of given volumetric capacity. This kind of fermentation (also referred to as "fed batch"), renders it possible to diminish the deleterious action of the inhibitors by keeping their concentration at a restricted level. However, the main drawback of this type of fermentation is that it is difficult to obtain high concentrations of microorganisms in the extracted material.
For instance, FR2554125 describes a method for preparing microorganism concentrates. However, the inventors have discovered that when using such a method, it was difficult to achieve high final concentrations of microorganisms without suffering from a major loss of biomass in the installation (pipelines of the apparatus, fermentor, harvesting equipments...). Indeed, microorganism-containing suspensions or concentrates are often very viscous and thus difficult to manipulate.

The suspension is then usually concentrated using techniques such as centrifugation, filtration, distillation, sedimentation or flocculation. This concentration step is often followed by freezing or freeze-drying or any known conservation techniques to preserve and/or store the microorganisms. The concentration step reduces the volume of the microorganism-containing suspension or concentrate to be treated for preservation and/or storage. By reducing the volume of the suspension or concentrate and increasing the concentration of the microorganisms in said suspension or concentrate, the costs can be reduced advantageously. The concentration step is very important on industrial scale processes, since efficient concentration of microbial suspensions is needed in order to reduce the costs associated with culture preservation, storage and transport.

Different techniques have been described in the art in order to provide microorganism concentrates with high yields. Typically, centrifugation techniques, such as ultracentrifugation, or filtration techniques such as microfiltration or ultrafiltration, are used in order to concentrate microorganism-containing suspensions.

Therefore, there is an unmet need in the art for methods for obtaining a microorganism concentrate having a desired concentration with a satisfactory yield (i.e. a limited loss of biomass in the installation).

### Summary of the invention

The inventors have discovered that addition of a monosaccharide and/or disaccharide and/or sugar alcohol to a microorganism-containing suspension before microorganism concentration leads to a surprising reduction in viscosity of said microorganism-containing suspension. When no monosaccharide and/or disaccharide and/or sugar alcohol is added to a microorganism-containing suspension before microorganism concentration, the viscosity of the concentrate is increased as the concentrate becomes pasty and difficult to transfer through the pipelines and the tanks.

The inventors have also discovered that addition of a monosaccharide and/or disaccharide and/or sugar alcohol to a microorganism-containing concentrate leads to a surprising reduction in viscosity of said microorganism-containing concentrate.

Hence the present invention relates to a method for reducing the viscosity of a microorganism-containing suspension or concentrate comprising the step of adding a monosaccharide and/or a disaccharide and/or a sugar alcohol to said microorganism-containing suspension or concentrate, after batch or continuous fermentation.

The present invention also relates to the use of a monosaccharide and/or disaccharide and/or sugar alcohol for reducing the viscosity of a microorganism-containing suspension or concentrate, after batch or continuous fermentation.

Also provided, is a method for reducing the viscosity of a microorganism-containing suspension or concentrate comprising the steps of:
a) adding a monosaccharide and/or a disaccharide and/or a sugar alcohol to a microorganism-containing suspension and/or to a microorganism-containing concentrate
b)concentrating said microorganism-containing suspension or said microorganism-containing concentrate, preferably by filtration and/or centrifugation, in order to obtain a microorganism-containing concentrate.

### Detailed description of the invention

### Method for reducing the viscosity of a microorganism-containing suspension or concentrate

The present invention relates to a method for reducing the viscosity of a microorganism-containing suspension or concentrate comprising the step of adding a monosaccharide and/or a disaccharide and/or a sugar alcohol to said microorganism-containing suspension or concentrate, after batch or continuous fermentation.

The present invention also relates to the use of a monosaccharide and/or a disaccharide and/or a sugar alcohol for reducing viscosity of a microorganism-containing suspension or concentrate, after batch or continuous fermentation.

As used herein, the term "viscosity" has its general meaning in the art. It refers to the measurement of the resistance of fluid which is being deformed by either shear stress or tensile stress. Viscosity can be expressed in Pa.s. Typically, the viscosity of a microorganism-containing suspension or concentrate can be measured by a flow measurement assay as described in the examples below. Typically, the viscosity of the concentrate can be measured according to the test A described in the examples below, using a Rheometer AR1000-N (Ta Instruments), at a temperature of 5°C, under a shear rate of 0 s⁻¹ to 200 s⁻¹.

Advantageously, the viscosity under standard conditions (at 5°C, under a shear rate of 0 s⁻¹ to 200 s⁻¹) of said microorganism-containing suspension or concentrate is significantly lower than the viscosity of a similar suspension or concentrate in which no monosaccharide, no disaccharide and no sugar alcohol has been added. Typically, the viscosity is reduced by a factor 1.5, 2, 3, 4, 5, 10, or even more compared to a microorganism-containing suspension or concentrate obtained without addition of monosaccharide and/or disaccharide and/or sugar alcohol.

In one embodiment, the invention also relates to the use of a monosaccharide and/or a disaccharide and/or a sugar alcohol for modifying the viscoelasticity of a microorganism-containing suspension or concentrate.
Viscoelasticity is the property of materials that exhibit both viscous and elastic characteristics when undergoing deformation. Viscous materials, like honey, resist shear flow and strain linearly with time when a stress is applied. Elastic materials strain instantaneously when stretched and just as quickly return to their original state once the stress is removed. Viscoelastic materials have elements of both of these properties and, as such, exhibit time dependent strain. Whereas elasticity is usually the result of bond stretching along crystallographic planes in an ordered solid, viscosity is the result of the diffusion of atoms or molecules inside an amorphous material.
Typically, the viscoelastic properties of a sample are assayed by an oscillation test B performed with a Rheometer, as illustrated in the examples below.
This test yields several response parameters, among which G' represents the elasticity and G" represents the viscosity.

According to one embodiment, the addition of a monosaccharide and/or a disaccharide and/or a sugar alcohol according to the invention results in a decrease of G" in an oscillation test B as described in the Examples below.
According to another embodiment, the addition of a monosaccharide and/or a disaccharide and/or a sugar alcohol according to the invention results in a decrease of both G" and G' in an oscillation test B as described in the Examples below.

### Method for obtaining a microorganism-containing concentrate

The invention also relates to a method for reducing the viscosity of a microorganism-containing suspension or concentrate comprising the steps of:
a) adding a monosaccharide and/or a disaccharide and/or a sugar alcohol to a microorganism-containing suspension and/or to a microorganism-containing concentrate;
b)concentrating said microorganism-containing suspension or said microorganism-containing concentrate in order to obtain a microorganism-containing concentrate ;
wherein steps a) and b) are performed either simultaneously or sequentially.

As used in the present invention, the terms "monosaccharide", "disaccharide" and "sugar alcohol" have their standard meaning in the art.
A monosaccharide according to the invention is a carbohydrate having the chemical formula C*ₓ*(H₂O)*_{y}*. In a preferred embodiment, the monosaccharide according to the invention is a hexose (where *x* is 6), i.e. a monosaccharide selected from the group consisting of allose, altrose, mannose, gulose, idose, galactose, talose, fructose, sorbose, tagadose. Suitable monosaccharides according to the invention can include both reducing monosaccharides and non-reducing monosaccharides. Preferred monosaccharides according to the invention are glucose, mannose, galactose, fructose and sorbose. In a preferred embodiment, said monosaccharide is fructose.

A disaccharide is the carbohydrate formed when two monosaccharides undergo a condensation reaction. Suitable disaccharides according to the invention can include both reducing disaccharides and non-reducing disaccharides. Typically, the disaccharide according to the invention can be selected from group consisting of sucrose, trehalose, cellobiose, lactose, maltose, lactulose, isomaltose, gentiobiose, isomaltose, laminaribiose, mannobiose and xylobiose. Preferred disaccharides according to the invention are sucrose, trehalose maltose, cellobiose and lactose. In a preferred embodiment, said disaccharide is sucrose.

A sugar alcohol, or polyol, is a hydrogenated form of carbohydrate whose carbonyl group has been reduced to a primary or secondary hydroxyl group. Typically, the sugar alcohol according to the invention can be selected from the group consisting of lactitol, sorbitol, mannitol, maltitol, glycol, glycerol, erythritol, threitol, arabitol, xylitol, ribitol, dulcitol, iditol, isomalt and polyglycitol. Preferred sugar alcohols according to the invention are lactitol, sorbitol, mannitol and maltitol.

In a preferred embodiment, the monosaccharide and/or disaccharide and/or sugar alcohol is selected from the group consisting of sucrose, fructose, trehalose, lactitol, maltitol and mannitol.
Even more preferably, the monosaccharide and/or disaccharide and/or sugar alcohol is sucrose.

In one embodiment of the invention, the monosaccharide and/or disaccharide and/or sugar alcohol is not a monosaccharide and/or disaccharide and/or sugar alcohol which can be metabolized by the microorganism.

Typically, the monosaccharide and/or disaccharide and/or sugar alcohol according to the invention can be added to the microorganism-containing suspension or concentrate as a solid powder or as a liquid form, for example a concentrated solution.

In a preferred embodiment, the monosaccharide and/or disaccharide and/or sugar alcohol is added to the microorganism-containing suspension or concentrate at a final concentration comprised between 10 and 120 g per kg, preferably between 30 to 100 g per kg, even more preferably between 50 to 80 g per kg of suspension or concentrate. In other words, the concentration of monosaccharide and/or disaccharide and/or sugar alcohol in the microorganism-containing suspension or concentrate can be comprised between 1 and 12% by weight, preferably between 3 and 10% by weight, even more preferably between 5 and 8% by weight.

The microorganism of the invention is typically selected from the group consisting of yeasts, molds, fungi, bacteria or any mixture thereof.
Examples of suitable yeasts are: *Kluyveromyces* spp, *Debaryomyces* spp, *Yarrowia* spp, *Pichia* spp, *Williopsis* spp, *Saccharomyces* spp.
Examples of suitable fungi/molds are: *Penicillium* spp, *Geotrichum* spp, *Lecanicillium* spp, *Trichothecium* spp.
Examples of suitable bacteria are: coryneform bacteria such as for example *Arthrobacter* spp, *Corynebacterium* spp, *Brevibacterium* spp; lactic acid bacteria; Micrococcaceae and bacteria of the *Staphylococcus* genus.
The microorganism of the invention is preferably a lactic acid bacterium. According to the invention, the term *"lactic acid bacterium"* includes any bacterium capable of producing, as the major metabolic end product of carbohydrate fermentation, lactic acid or at least one of its derivatives (including, but not limited to, propionic acid). The term is therefore intended to include propionic acid bacteria (PAB), which produce propionic acid as a carbohydrate fermentation product.
Preferably the bacteria according to the present invention are lactic acid bacteria which are generally recognized as safe for animal or human consumption (i.e. GRAS approved).
Suitable bacteria may be selected from the genus *Lactococcus, Lactobacillus, Leuconostoc, Bifidobacterium, Carnobacterium, Enterococcus, Propionibacterium, Pediococcus, Streptococcus* and mixtures thereof.
Typically, the microorganisms are probiotics or DFM (Direct Fed Microbials). According to the invention *"probiotics"* or "DFMs" means live microorganisms which when administered in adequate amounts confer a health benefit on the host, the host being a human in the case of probiotics and an animal in the case of DFMs. The probiotic microorganisms or DFMs most commonly used are principally bacteria and yeasts of the following genera: *Lactobacillus spp., Streptococcus spp., Enterococcus spp., Bifidobacterium spp.* and *Saccharomyces spp.*
In a preferred embodiment, the microorganism is a bacterium, preferably a bacterium selected from the group consisting of bacteria from the *Acetobacter, Bifidobacterium, Carnobacterium, Enterococcus, Lactococcus, Lactobacillus, Leuconostoc, Pediococcus, Oenococcus, Propionibacterium,* and/or *Streptococcus* genus.

In a preferred embodiment, said bacterium is a bacterium selected from the group consisting of bacteria from the *Lactococcus, Lactobacillus, Leuconotoc, Bifibobacterium, Pediococcus,* and/or *Streptococcus* genus.

In one embodiment of the invention, the microorganism is an exopolysaccharide-producing strain of lactic acid bacterium. Indeed, certain bacteria secrete high levels of exopolysaccharides under certain fermentation conditions. Without wishing to be bound to theory, it is thought that these exopolysaccharides contribute to increasing the viscosity of suspensions or concentrates containing such bacteria. The use of a monosaccharide and/or disaccharide and/or sugar alcohol according to the invention is therefore particularly advantageous for these strains of bacteria.

According to the method of the invention, steps a) and b) can be performed either simultaneously or sequentially.
Without wishing to be bound by theory, it is believed that adding a monosaccharide and/or a disaccharide and/or a sugar alcohol to a microorganism-containing suspension and/or to a microorganism-containing concentrate helps to reduce the viscosity and/or viscoelasticity of said microorganism-containing suspension and/or concentrate by fragilizing the structure of said microorganism-containing suspension and/or concentrate. The resulting treated microorganism-containing suspension can be easily more concentrated (e.g. into a centrifugation or ultrafiltration equipment). In addition the resulting microorganism-containing suspension and/or concentrate is easier to manipulate. Hence, it can be processed through installation (pipelines of the apparatus, fermentor, harvesting equipments...) with minimal loss of biomass. Another advantage of adding a monosaccharide and/or a disaccharide and/or a sugar alcohol to a microorganism-containing suspension and/or concentrate is to permit or enhance the formation of drops of said suspension or concentrate when frozen pellets form is desired.
To the inventors' knowledge these particular kinds of sugars are known to be used as growth medium components to be fermented by microorganisms and/or as protectant agents before a preservation step to stabilize microorganisms, but they were never used at an intermediate step for decreasing the rheological properties (viscosity and/or viscoelasticity) of a microorganism-containing suspension and/or concentrate, and therefore reducing loss of biomass in the installation.

As used herein, the expressions "microorganism-containing suspension" and "microorganism-containing concentrate" refer to a continuous spectrum of liquids containing increasing amounts of microorganisms. A "microorganism-containing suspension" generally refers to a suspension containing relatively low concentrations of microorganisms which are not suitable for transport or storage. Typically, a microorganism-containing suspension is obtained directly after the fermentation step without any concentration step and contains up to 40 g microorganism per kg of suspension.
A "microorganism-containing concentrate" refers to a suspension which has been through at least one concentration step and generally contains a concentration of microorganisms (or Cx) greater than 40 g/kg. It is desirable to obtain microorganism-containing concentrate having a concentration (or Cx) of at least 45 g/kg, preferably at least 50, 60, 70, 80, 90, 100, 110, 120, 130, 140, 150, 160, 170, 180, 190, 200, 210, 220 g/kg.

Obtaining of a "concentrate" from a "suspension" can be carried out by several methods, which all enrich the liquid in microorganisms relative to the other components of the liquid. Techniques such as centrifugation, filtration, distillation, sedimentation and/or flocculation can be used. Preferably, concentration is carried out by centrifugation and/or filtration techniques, known in the art.

As used herein, the term "centrifugation" refers to a method of separating immiscible liquids or solids from liquids through application of centrifugal force. In some preferred embodiments, the separation methods involve subjecting a fluid-containing mixture to a high gravitational force (g). Upon application of this centrifugal force (often many times the force of gravity [xg]), the different components present in the mixture are separated. In some preferred embodiments of the present invention, centrifugation is applied to a liquid which contains microbial cells. At the completion of the centrifugation process, the microbial cells are located in the "concentrate" (i.e., the more "solid" portion of the product) and the liquid is the "supernatant" (or "eluate"). In some preferred embodiments, the liquid portion contains no or very few microorganisms. In industrial scale volumes, the concentrate typically contains from between about 5% and about 20% solids.

In some preferred embodiments, centrifugation is carried out in a centrifuge that provides a gravitational force from about 400 to about 65000 xg (*i.e.,* times gravity), while in other embodiments, the gravitational force is from about 4000 to 20000 xg, and in other embodiments, the gravitational force is from about 6000 to about 10000 xg. However, it is not intended that the present invention be limited to any particular centrifuge or gravitational force. In some further embodiments of the present invention the centrifugation step is repeated between about two and about four times. In some particular embodiments, the centrifugation step is repeated twice. However, it is not intended that the present invention be limited to any particular number of repetitions of the centrifugation step, as any suitable number of repetitions will find use. In some embodiments, the concentrate obtained is placed in solution prior to each centrifugation step (*e.g.,* by adding any suitable liquid, such as water or food grade buffer). It is not intended that the present invention be limited to any particular liquid.

As used herein, the term "filtration" refers to a separation process consisting of passing a solution through a filtration membrane to separate the components within the liquid, based on the size of the components. The filtration membrane has pores of a given size designed to retain components that are larger than the pore size, but allow components that are smaller than the pore size to pass through the membrane. Thus, in some preferred embodiments, the solution contains solid elements (*e.g.*, microorganisms) that are larger than the pores of the filtration membrane. In these embodiments, the microorganisms are present in the "concentrate" (or "retentate") and the liquid phase that passes through the membrane is referred to as "permeate" or "filtrate". In addition to containing liquid, in some embodiments, the permeate also contains other components. In "conventional filtration" the separation is carried out due to natural gravitational pressure, while in "pressure filtration," additional pressure (*e.g.*, greater pressure on the concentrate side and/or a depression on the permeate side) helps to accelerate the filtration process. Any suitable filtration methods find use in the present invention, including but not limited to microfiltration and ultrafiltration. However, in some particularly preferred embodiments, ultrafiltration is used.

As used herein, the term "microfiltration" refers to any filtration method that involves use of microporous filtration membranes. The pore size of these microfiltration membranes is usually about 0.1 µm to about 10 µm. The micro filtration membranes used in the methods of the present invention typically have a molecular weight cut-off of about 350 kDa to about 5 000 kDa.

As used herein, the term "ultrafiltration" refers to any filtration method using filtration membranes having smaller pore sizes than those used for microfiltration, usually about 0.01 µm to about 0.1 µm. The ultrafiltration membranes used in the methods of the present invention typically have a molecular weight cut-off of about 5 kDa to about 350 kDa.

In some methods of the present invention, the filtration step is accomplished using microfiltration with a microfiltration membrane having a pore size from about 0.1 to about 10 µm, while in other embodiments the pore size is from about 0.1 to about 5µm, and in still other embodiments, the pore size is from about 0.1 to about 2 µm. In some preferred embodiments, the microfiltration membrane has a molecular weight cut-off of about 350 kDa to about 5 000 kDa, while in other embodiments, the molecular weight cut-off is about 400 kDa to about 1 000 kDa, and in still other embodiments, the molecular weight cut-off is about 500 kDa.

In some alternative methods of the present invention, the filtration step is accomplished using ultrafiltration with an ultrafiltration membrane having a pore size from about 0.01 µm to about 0.1 µm, while in other embodiments, the pore size is from about 0.02 to about 0.1 µm, and in still other embodiments, the pore size is from about 0.05 to about 0.1 µm. In some embodiments, the ultrafiltration membrane has a molecular weight cut-off of about 5 kDa to about 350 kDa, while in other embodiments, the molecular weight cut-off of is from about 30 kDa to about 200 kDa, and in still further embodiments, the molecular weight cut-off is about 150 kDa.

It is intended that any suitable filtration method will find use in the present invention, including but not limited to conventional filtration methods (e.g., by use of gravitational force) and tangential or cross-flow filtration methods. The term "cross flow filtration" and "tangential filtration" are used interchangeably herein in reference to any filtration method wherein the concentrate continuously and tangentially flows across the surface of the filtration membrane while the permeate flows trough the filtration membrane.

In some embodiments of the present invention the filtration step is repeated between about two and about four times.

One or several intermediate washing step(s) can also be included in the process. Such a step is particularly interesting when a subsequent step of conservation is present, especially a freeze-drying step, in order to improve the conservation of microorganisms.

In a preferred embodiment the monosaccharide and/or disaccharide and/or sugar alcohol is added to a microorganism-containing suspension or concentrate into the bioreactor, i.e. prior to any transfer into other vessels (e.g. a tank wherein a protectant is added, or a tank containing already a protectant) or prior to microorganism recovery or prior to microorganism concentration. However it is not intended to be used as a growth medium component (nutrient substrate) for the microorganisms, so it is generally added once the fermentation of the microorganism-containing suspension is over.

The present invention can be implemented within a wide range of existing culture processes. In addition steps a) and b) can be performed either simultaneously or sequentially. By the term "sequentially", it shall be understood that both orders of sequences are encompassed, i.e. step a) is before step b) or step b) is before step a).

In one embodiment, the monosaccharide and/or disaccharide and/or sugar alcohol can be added to a microorganism-containing suspension prior to its concentration, i.e. step a) occurs before step b). This is typically the case of batch fermentation process, when the concentration step is carried out by centrifugation, since the microorganism-containing suspension has to be transferred out of the bioreactor into a centrifugation vessel before being concentrated into a microorganism containing concentrate.

In another embodiment, the monosaccharide and/or disaccharide and/or sugar alcohol can be added to a microorganism-containing concentrate, i.e. once the concentration step has already been carried out, i.e. step a) occurs after step b). This is typically the case of continuous fermentation process, when the concentration step is carried out by filtration in the same bioreactor as the fermentation step, without any transfer of the fermentate.

Alternatively, steps a) and b) can be carried out simultaneously, e.g. when the concentration is carried out by filtration. Indeed, the filtration can be a continuous fermentation process involving several cycles. The monosaccharide and/or disaccharide and/or sugar alcohol can therefore be added in increasing concentrations to the microorganism-containing suspension or concentrate, as the concentration of microorganism in said microorganism-containing suspension or concentrate increases.

In a preferred embodiment, step a) occurs before step b), so preferably the invention relates to a method for reducing the viscosity of a microorganism-containing suspension or concentrate comprising the steps of:
a) adding a monosaccharide and/or a disaccharide and/or a sugar alcohol to a microorganism-containing suspension
b) concentrating said microorganism-containing suspension, preferably by centrifugation, in order to obtain a microorganism-containing concentrate

As used herein, the term "fermentation" or "industrial fermentation" has its general meaning in the art. It refers to the aerobic and/or anaerobic and/or micro-aerobic growth of microorganisms such as for example bacteria and fungi in order to produce large amounts of biomass, which are then used to make products useful to humans.
Typically, in the methods according to the invention, steps a) and b) are carried out once the fermentation of the microorganism-containing suspension is over.
The skilled person in the art knows when the fermentation is considered to be over, i.e., when the growth of the microorganisms in the suspension (or fermentate) substantially stops.
Typically, the fermentation step ends once there is no longer any substrate to be metabolized by said microorganisms in order to support their growth.
Typically, in batch fermentation process, the fermentation step can be considered to be over when the concentration of microorganisms in the suspension reaches a certain threshold.
Typically, the fermentation step can be considered to be over when the quantity of microorganisms reaches a threshold of 10E8-10E12 cfu/mL. Alternatively, the fermentation step can be considered to be over when the optical density at 550-700 nm of the microorganism-containing suspension is stabilized to a maximum value (from 1 to 20).
If the fermentation is carried out in a fed-batch process, the fermentation step ends when the supply of nutrient substrate is stopped.

In one embodiment, the nutrient substrate (carbon source) during the fermentation step is not a monosaccharide and/or a disaccharide and/or a sugar alcohol.

The microorganism-containing suspension can be obtained by different known culture processes.
In some embodiments, the microorganism-containing suspension is obtained by a continuous fermentation process, such as a continuous fermentation process with elimination, that is to say a process combining a continuous cultivation of microorganisms with elimination of inhibitors and recycling of microorganisms within the reactor while biomass grows.
In a preferred embodiment, the microorganism-containing suspension is obtained by a batch process.

Typically, the fermentation step can be carried out at a temperature between 20°C and 45°C. Once the fermentation step is over, the fermentate or microorganism-containing suspension can be cooled to a temperature between 5°C and 25°C.

In some embodiments, the concentration step b) is carried out by centrifugation, filtration, distillation, sedimentation and/or flocculation, preferably by centrifugation and/or filtration. In a preferred embodiment, the concentration step b) is carried out by centrifugation.

In one embodiment, the method of the invention further comprises the following steps:
c) optionally adding a protectant to the microorganism-containing concentrate obtained after steps a) and b);
d) conserving said microorganism-containing concentrate by cooling, freezing, drying and/or freeze-drying.

A suitable protectant (also called cryoprotectant or lyoprotectant) can be chosen amongst any well-known agents such as sucrose, trehalose, milk solids, maltodextrine, cyclodextrin, yeast extract, maltitol, spray gum, fish gelatine... The role of such agent is to protect the microorganisms while being submitted to the conservation step d).
In a specific embodiment of the present invention, a monosaccharide and/or a disaccharide and/or a sugar alcohol is added under step b) for reducing the viscosity of a microorganism-containing suspension or concentrate, then another one (that can be the same or a different one) is added as a protectant at step c) before conservation step d).

The conservation step d) can be carried out by standard methods known in the art.
Typically, cooling is obtained by bringing the concentrate to a temperature comprised between 4°C and 25°C; freezing is obtained by bringing the concentrate to a temperature below 0°C, preferably below -18°C, even more preferably around -80°C, even more preferably around -196°C. The freezing can be performed in blocks of any form or can be obtained by dropping the suspension or concentrate into liquid nitrogen in order to obtain frozen pellets.
Drying can be obtained by vacuum-drying, whereby the concentrate is dehydrated by evaporation of the water, under vacuum at a temperature above 0°C. Drying can also be obtained by absorption of microorganisms-containing concentrate on a porous inert carrier. Another suitable example of drying is spray-drying, also called atomisation that can be performed using a spray dryer or a fluid-bed dryer.
Freeze-drying, also called lyophilization, is a conservation process whereby the material is frozen (blocks, drops, pellets...) below 0°C, preferably below -18°C, even more preferably around -80°C, even more preferably around -196°C and the surrounding pressure is then reduced and enough heat is added to allow the frozen water in the material to sublime directly from the solid phase to the gas phase. In certain embodiments, the material is frozen in the freeze-dryer. In some other embodiments, the material is introduced directly under a frozen form into the freeze-dryer.

In one embodiment, the present invention provides a method for preparing microorganisms concentrates comprising at least the following steps:
- obtaining a microorganism-containing suspension by a batch fermentation process;
- adding a monosaccharide and/or a disaccharide and/or a sugar alcohol to a microorganism-containing suspension and/or concentrate (step a))
- concentrating said microorganism-containing suspension and/or concentrate preferably by centrifugation (step b)) ;
- recovering said microorganism-containing concentrate or transferring it into another vessel
- optionally submitting it to a conservation step such as cooling, freezing, drying and/or freeze-drying after optional addition of a protectant.

In this embodiment, steps a) and b) are performed either simultaneously or sequentially. Preferably steps a) and b) are simultaneous or step b) occurs after step a). More preferably step b) occurs after step a).
So in a preferred embodiment, the present invention provides a method for preparing microorganisms concentrates comprising at least the following steps:
- obtaining a microorganism-containing suspension by a batch fermentation process;
- adding a monosaccharide and/or a disaccharide and/or a sugar alcohol to said microorganism-containing suspension (step a))
- concentrating said microorganism-containing suspension, preferably by centrifugation (step b));
- recovering said microorganism-containing concentrate or transferring it into another vessel
- optionally submitting it to a conservation step such as cooling, freezing, drying and/or freeze-drying after optional addition of a protectant.

### Microorganism-containing concentrates of the invention

Also described is a microorganism-containing concentrate obtainable by steps a) and b) of the methods described above.

In a preferred embodiment, the concentration of microorganisms in said microorganism-containing concentrate is superior to 80, 90, 130 g/kg, preferably greater than 140, 150, 160, 170, 180, 190, 200, 210, 220 g/kg.
Advantageously, the concentrates according to the invention are less viscous than concentrates having similar concentrations of microorganisms in the absence of monosaccharides or disaccharides or sugar alcohol.
Typically, for a concentration between 100 and 220 g/kg of microorganism, the viscosity of the concentrates according to the invention measured according to test A is comprised between 0.05 and 30 Pa.s.

Typically, the concentration of microorganisms in said microorganism-containing concentrate is at least 10⁹ colony-forming units (cfu) per g, preferably at least 10¹⁰ cfu/g, even more preferably at least 10¹¹ cfu/g, at least 10¹² cfu/g, at least 10¹³ cfu/g.

Advantageously, the method of the invention enables the obtaining of highly concentrated microorganism-containing concentrates, with high yields (reduced loss of biomass within the installation).

Other features and advantages of the invention will emerge upon reading the following nonlimiting examples.

### EXAMPLES

### Material and methods

### Microorganisms

Different types of microorganisms were used for these studies.
The following strains from the Danisco Global Culture Collection were used:
*Streptococcus thermophilus* (Example 1)
*Lactococcus lactis subspecies lactis* (Example 2); and
*Geotrichum candidum* (Example 3).

### Fermentation process

The *Streptococcus thermophilus* bacterium was grown in bioreactors according to the continuous fermentation process with elimination as described in Cabau et al., FR2554125.
The *Lactococcus lactis subspecies lactis* bacterium was grown in bioreactors according to a batch fermentation process.
The fungus *Geotrichum candidum* was grown according to aerobic batch fermentation process.

### Concentration

For *Streptococcus thermophilus,* the suspension was concentrated by ultrafiltration. Sucrose was added after the concentration step.
For *Lactococcus lactis subspecies lactis,* the suspension was concentrated by centrifugation and sucrose was added either before or after the concentration step.
For *Geotrichum candidum,* sucrose was added after the concentration by centrifugation of a batch fermentate.

### Rheological tests

Flow measurements, under a gradient of shear rates, were carried out using a Rheometer AR1000-N from Ta Instruments, as described below.
Further, the viscoelastic rheological properties of certain suspensions or concentrates were studied using the same Rheometer, under "oscillation test" conditions, as described below.

**Flow measurements (Test A):**

| | |
|---|---|
| Geometry | Steel conical concentric cylinder + external water jacket |
| | Inertia 13.02 N.m.S2, R2 15mm, R1 13.83mm, H 32mm, R2/R1 1.0845 |
| | Gap 4000µm |
| Measurement protocol | Shear rate sweep by linear mode |
| | Logarithmic ramp of shear rate from 0s-1 to 200 s⁻¹ |
| | Duration: 3' |
| | Temperature measurement: +5° |
| Parameters studied | Viscosity η at 0, 10, 64 and 200 s⁻¹ |
| | Flow curves: η=f(γ) |

**Visco-elastic measurements (oscillation test - Test B);**

| | |
|---|---|
| Geometry | Steel plane/plane |
| | Inertia 7.002N.m.S2, diameter 40mm |
| | Gap 500µm |
| | Temperature control by Peltier plane |
| Measurement protocol | Determination of linear visco-elastic region (RVE) by shear stress sweep Logarithmic ramp of shear stress from 0.01 Pas to 15 Pa in 35 points |
| | Frequency: 1 Hz |
| | Temperature measurement: +5° |
| Parameters studied | Critical shear stress value (end of RVE)* |
| | G', G" at 1 Pa |

### Example 1: Streptococcus thermophilus

The following flow measurements were obtained with the *S. thermophilus* strain (Table 1A):

| | | **Cx (g/kg)** | **% sucrose** | **Flow measurement at +5°C** | | | |
|---|---|---|---|---|---|---|---|
| | | | | **Initial η** | **η 10 s⁻¹** | **η 64 s⁻¹** | **η 200 s⁻¹** |
| **1** | **Concentrate** | 90 | 0% | 2.226 | 0.520 | 0.103 | 0.047 |
| **2** | **Concentrate** | 130 | 0% | 18.410 | 4.402 | 0.577 | 0.265 |
| **3** | **Concentrate +sucrose** | 130 | 11.90% | 1.814 | 0.810 | 0.175 | 0.081 |
| | | | | 1.749 | 0.742 | 0.168 | 0.079 |
| **4** | **Concentrate+sucrose** | 130 | 9.50% | 4.114 | 1.291 | 0.232 | 0.103 |
| **5** | **Concentrate+sucrose** | 130 | 5.95% | 7.085 | 1.965 | 0.320 | 0.137 |
| **6** | **Concentrate+sucrose** | 130 | 2.38% | 10.900 | 2.826 | 0.422 | 0.180 |

Typically, the initial viscosity of a concentrate containing 130 g/kg of the *Streptococcus thermophilus* strain was reduced:
- by a factor of about 2 by the addition of 2.38% sucrose (compare samples 2 and 6)
- by a factor of about 10 by the addition of 11.9% sucrose (compare samples 2 and 3).

In addition, the addition of sucrose modified the viscoelastic properties of the suspension and/or concentrate containing the *Streptococcus thermophilus* strain (see Table 1B):

| | | **Cx (g/kg)** | **% sucrose** | **Oscillation test at +5°C** | |
|---|---|---|---|---|---|
| | | | | **G' at 1Pa (Pa)** | **G" at 1Pa (Pa)** |
| **1** | **Concentrate** | 90 | 0% | 29.83 | 1.774 |
| | | | | 31.19 | 1.963 |
| **2** | **Concentrate** | 130 | 0% | 147.3 | 7.67 |
| | | | | 152.3 | 8.089 |
| **3** | **Concentrate + sucrose** | 130 | 11.90% | 11.49 | 2.610 |
| | | | | 8.497 | 2.333 |
| **4** | **Concentrate+sucrose** | 130 | 9.50% | 20.18 | 3.60 |
| **5** | **Concentrate+sucrose** | 130 | 5.95% | 38.56 | 5.05 |
| **6** | **Concentrate+sucrose** | 130 | 2.38% | 63.77 | 6.994 |
| | | | | 64.67 | 6.949 |

As can be seen in Table 1B, addition of sucrose led to a diminution of both the viscosity component (G", also called loss modulus) and the elastic component (G' also called the storage modulus)

The inventors repeated the experiments with fructose, trehalose, lactitol, maltitol and mannitol instead of sucrose. The addition of these monosaccharides or disaccharides or sugar alcohol also led to a reduction of the viscosity and to modifications of the visco-elastic properties of the concentrates.

Finally, the effect of these modifications on the yield of the production process at industrial scale was evaluated.

Addition of sucrose significantly increased the biomass recovery within the installation. Indeed, the yield was increased from 75% in the absence of sucrose to 95% when sucrose was added after the concentration step.
The acidification properties of the resulting concentrate were not affected.

Hence addition of a monosaccharide or disaccharide or sugar alcohol to the microorganism-containing concentrate results in an increased yield, without affecting the quality of the concentrate.

Additional experiments were performed, wherein sucrose was added before and/or during the concentration step. Such trials similarly demonstrated a viscosity decrease of the final concentrate in comparison with the same concentrate without any sucrose addition.

### Example 2: Lactococcus lactis

The effect of sucrose was also tested on a bacterium from a different genus: Lactococcus (Table 2A below).

| | **Cx (g/kg)** | **Flow measurement at +5°C** | | | |
|---|---|---|---|---|---|
| | | **Initial η** | **η 10 s⁻¹** | **η 64 s⁻¹** | **η 200 s⁻¹** |
| **1 Concentrate control** | **200** | **3.497** | **1.755** | **1.818** | **0.8772** |
| **2 Concentrate+10%sucrose** | **200** | **0.09041** | **0.0788** | **0.1116** | **0.1471** |
| **3 Concentrate+5%sucrose** | **200** | **1.405** | **0.5416** | **0.6344** | **0.5778** |
| **4 Concentrate+10%sucrose** | **200** | **0.097** | **0.08251** | **0.1115** | **0.1528** |

In sample 2 addition of sucrose powder was made on the cell concentrate obtained after centrifugation.
In samples 3 and 4 the addition of sucrose was made at the fermentate level after final growth and biomass production using respectively 10% and 20% of a 50% sucrose syrup equivalent to 5% and 10% final sucrose concentration.

### Example 3: Geotrichum candidum

A fungus, Geotrichum, was produced by an aerobic batch fermentation process and concentrated by centrifugation. Sucrose was added at the end of the concentration step.

The following flow measurements were observed (Table 3A):

| | | **% sucrose** | **Flow measurement at +5°C** | | | |
|---|---|---|---|---|---|---|
| | | | **Initial η** | **η 10 s⁻¹** | **η 64 s⁻¹** | **η 200 s⁻¹** |
| **1** | **Concentrate control** | 0% | 7.334 | 3.612 | 1.993 | 1.094 |
| **2** | **Concentrate+10%sucrose** | 10% | 3.577 | 1.901 | 0.9657 | 0.466 |

The following viscoelastic properties were observed (Table 3B):

| | | **% sucrose** | **Oscillation test at +5°C** | |
|---|---|---|---|---|
| | | | **G' at 1Pa (Pa)** | **G" at 1Pa (Pa)** |
| **1** | **Concentrate** | 0% | 62.79 | 16.74 |
| | | | 128.6 | 27 |
| **2** | **Concentrate + sucrose** | 10% | 37.62 | 9.507 |
| | | | 48.39 | 11.32 |

Addition of sucrose had an effect of both components G' and G" of the viscoelasticity.

### Conclusion

The above results demonstrate that addition of a monosaccharide and/or disaccharide and/or a sugar alcohol can reduce the viscosity of a microorganism-containing suspension or concentrate. Said monosaccharide and/or disaccharide and/or sugar alcohol also modifies the viscoelastic properties of microorganism-containing suspension or concentrates.

## Claims

1. Use of a monosaccharide and/or disaccharide and/or sugar alcohol for reducing the viscosity of a microorganism-containing suspension or concentrate, after batch or continuous fermentation.

2. Method for reducing the viscosity of a microorganism-containing suspension or concentrate comprising the step of adding a monosaccharide and /or a disaccharide and/or a sugar alcohol to said microorganism-containing suspension or concentrate, after batch or continuous fermentation.

3. Use according to claim 1 or method according to claim 2, wherein said monosaccharide and/or disaccharide and/or sugar alcohol modifies the viscoelasticity of said microorganism-containing suspension or concentrate.

4. Method for reducing the viscosity of a microorganism-containing suspension or concentrate comprising the steps of:
a) adding a monosaccharide and/or a disaccharide and/or a sugar alcohol to a microorganism-containing suspension and/or to a microorganism-containing concentrate;
b) concentrating said microorganism-containing suspension and/or said microorganism-containing concentrate in order to obtain a microorganism-containing concentrate,
wherein steps a) and b) are performed either simultaneously or sequentially.

5. Use or method according to any of the preceding claims, wherein the monosaccharide and/or disaccharide and/or sugar alcohol is selected from the group consisting of sucrose, fructose, trehalose, lactitol, maltitol and mannitol, preferably sucrose.

6. Use or method according to any of the preceding claims wherein the monosaccharide and/or disaccharide and/or sugar alcohol is added to the microorganism-containing suspension or concentrate at a final concentration comprised between 10 and 120 g per kg, preferably between 30 to 100 g per kg, even more preferably between 50 to 80 g per kg of suspension or concentrate.

7. Use or method according to any of the preceding claims wherein the microorganism is selected from the group consisting of yeasts, molds, fungi, bacteria or any mixture thereof and preferably a bacterium selected from the group consisting of bacteria from the *Lactococcus, Lactobacillus, Leuconotoc, Bifibobacterium, Pediococcus,* and/or *Streptococcus* genus.

8. Method according to claim 4 to 7, wherein step b) is performed by centrifugation and/or filtration such as ultrafiltration.

9. Method according to any one of claims 4 to 8, wherein the concentration step b) is carried out by centrifugation.

10. Method according to claim 4 to 9, wherein steps a) and b) are carried out once the fermentation of the microorganism-containing suspension is over and/or prior to any transfer into other vessels.

11. Method according to any one of claims 4 to 10, further comprising the following steps:
c) optionally adding a cryoprotectant to the microorganism-containing concentrate obtained after steps a) and b);
d) conserving said microorganism-containing concentrate by cooling, freezing, drying and/or freeze-drying.

12. Method according to any one of claims 4 to 11, wherein said method comprises at least the following steps:
- obtaining a microorganism-containing suspension by a batch fermentation process;
- adding a monosaccharide and/or a disaccharide and/or a sugar alcohol to said microorganism-containing suspension (step a) ;
- concentrating said microorganism-containing suspension, preferably by centrifugation (step b)
- recovering said microorganism-containing concentrate or transferring it into another vessel
- optionally submitting it to a conservation step such as cooling, freezing, drying and/or freeze-drying.

## Patentansprüche

1. Verwendung eines Monosaccharids und/oder Disaccharids und/oder Zuckeralkohols zur Verringerung der Viskosität einer Suspension bzw. eines Konzentrats, die bzw. das Mikroorganismen enthält, nach diskontinuierlicher oder kontinuierlicher Fermentation.

2. Verfahren zur Verringerung der Viskosität einer Suspension bzw. eines Konzentrats, die bzw. das Mikroorganismen enthält, umfassend den Schritt der Zugabe eines Monosaccharids und/oder eines Disaccharids und/oder eines Zuckeralkohols zur Suspension bzw. zum Konzentrat, die bzw. das Mikroorganismen enthält, nach diskontinuierlicher oder kontinuierlicher Fermentation.

3. Verwendung nach Anspruch 1 oder Verfahren nach Anspruch 2, wobei das Monosaccharid und/oder Disaccharid und/oder der Zuckeralkohol die Viskoelastizität der Suspension bzw. des Konzentrats, die bzw. das Mikroorganismen enthält, modifiziert.

4. Verfahren zur Verringerung der Viskosität einer Suspension bzw. eines Konzentrats, die bzw. das Mikroorganismen enthält, umfassend die Schritte:
a) Zugabe eines Monosaccharids und/oder eines Disaccharids und/oder eines Zuckeralkohols zu einer Mikroorganismen enthaltenden Suspension und/oder zu einem Mikroorganismen enthaltenden Konzentrat;
b) Konzentrieren der Mikroorganismen enthaltenden Suspension und/oder des Mikroorganismen enthaltenden Konzentrats, um ein Mikroorganismen enthaltendes Konzentrat zu erhalten,
wobei Schritt a) und b) entweder gleichzeitig oder nacheinander erfolgen.

5. Verwendung oder Verfahren nach einem der vorhergehenden Ansprüche, wobei das Monosaccharid und/oder Disaccharid und/oder der Zuckeralkohol aus der Gruppe bestehend aus Saccharose, Fructose, Trehalose, Lactit, Maltit und Mannit, vorzugsweise Saccharose ausgewählt ist.

6. Verwendung oder Verfahren nach einem der vorhergehenden Ansprüche, wobei das Monosaccharid und/oder Disaccharid und/oder der Zuckeralkohol zur Suspension bzw. zum Konzentrat, die bzw. das Mikroorganismen enthält, in einer Endkonzentration gegeben wird, die zwischen 10 und 120 g pro kg, bevorzugt zwischen 30 und 100 g pro kg, noch stärker bevorzugt zwischen 50 und 80 g pro kg Suspension bzw. Konzentrat liegt.

7. Verwendung oder Verfahren nach einem der vorhergehenden Ansprüche, wobei der Mikroorganismus aus der Gruppe bestehend aus Hefen, Schimmelpilzen, Pilzen, Bakterien oder einem beliebigen Gemisch davon und vorzugsweise einem aus der Gruppe bestehend aus Bakterien aus der Gattung *Lactococcus, Lactobacillus, Leuconotoc, Bifidobacterium, Pediococcus* und/oder *Streptococcus* ausgewählten Bakterium ausgewählt ist.

8. Verfahren nach Anspruch 4 bis 7, wobei Schritt b) über Zentrifugation und/oder Filtration, wie etwa Ultrafiltration, erfolgt.

9. Verfahren nach einem der Ansprüche 4 bis 8, wobei der Konzentrationsschritt b) mittels Zentrifugation durchgeführt wird.

10. Verfahren nach Anspruch 4 bis 9, wobei Schritt a) und b) nach Beendigung der Fermentation der Mikroorganismen enthaltenden Suspension und/oder vor einer Überführung in andere Gefäße durchgeführt werden.

11. Verfahren nach einem der Ansprüche 4 bis 10, ferner umfassend die folgenden Schritte:
c) gegebenenfalls Zugabe eines Gefrierschutzstoffs zum nach Schritt a) und b) erhaltenen Mikroorganismen enthaltenden Konzentrat;
d) Konservieren des Mikroorganismen enthaltenden Konzentrats durch Kühlen, Einfrieren, Trocknen und/oder Gefriertrocknen.

12. Verfahren nach einem der Ansprüche 4 bis 11, wobei das Verfahren wenigstens die folgenden Schritte umfasst:
- Gewinnen einer Mikroorganismen enthaltenden Suspension mit einem diskontinuierlichen Fermentationsverfahren;
- Zugabe eines Monosaccharids und/oder eines Disaccharids und/oder eines Zuckeralkohols zur Mikroorganismen enthaltenden Suspension (Schritt a);
- Konzentrieren der Mikroorganismen enthaltenden Suspension, vorzugsweise durch Zentrifugation (Schritt b)
- Gewinnen des Mikroorganismen enthaltenden Konzentrats oder Überführen davon in ein anderes Gefäß
- gegebenenfalls Durchführen eines Konservierungsschritts damit, wie etwa Kühlen, Einfrieren, Trocknen und/oder Gefriertrocknen.

## Revendications

1. Utilisation d'un monosaccharide et/ou d'un disaccharide et/ou d'un alcool de sucre, afin de réduire la viscosité d'une suspension ou d'un concentré contenant des microorganismes, après une fermentation en mode discontinu ou continu.

2. Méthode destinée à la réduction de la viscosité d'une suspension ou d'un concentré contenant des microorganismes, comprenant l'étape consistant à ajouter un monosaccharide et/ou un disaccharide et/ou un alcool de sucre à ladite suspension ou audit concentré contenant des microorganismes, après une fermentation en mode discontinu ou continu.

3. Utilisation selon la revendication 1 ou méthode selon la revendication 2, dans laquelle ledit monosaccharide et/ou disaccharide et/ou alcool de sucre modifie la viscoélasticité de ladite suspension ou dudit concentré contenant des microorganismes.

4. Méthode destinée à la réduction de la viscosité d'une suspension ou d'un concentré contenant des microorganismes, comprenant les étapes consistant à :
a) ajouter un monosaccharide et/ou un disaccharide et/ou un alcool de sucre à une suspension contenant des microorganismes et/ou à un concentré contenant des microorganismes ;
b) concentrer ladite suspension contenant des microorganismes et/ou ledit concentré contenant des microorganismes, afin d'obtenir un concentré contenant des microorganismes ;
où les étapes a) et b) sont réalisées simultanément ou bien séquentiellement.

5. Utilisation ou méthode selon l'une quelconque des revendications précédentes, dans laquelle le monosaccharide et/ou le disaccharide et/ou l'alcool de sucre est choisi dans le groupe constitué par le saccharose, le fructose, le tréhalose, le lactitol, le maltitol et le mannitol, préférablement le saccharose.

6. Utilisation ou méthode selon l'une quelconque des revendications précédentes, dans laquelle le monosaccharide et/ou le disaccharide et/ou l'alcool de sucre est ajouté à la suspension ou au concentré contenant des microorganismes selon une concentration finale comprise entre 10 et 120 g par kg, préférablement allant de 30 à 100 g par kg, encore plus préférablement allant de 50 à 80 g par kg de suspension ou de concentré.

7. Utilisation ou méthode selon l'une quelconque des revendications précédentes, dans laquelle le microorganisme est choisi dans le groupe constitué par les levures, les moisissures, les champignons, les bactéries ou un mélange quelconque de ceux-ci, et préférablement une bactérie choisie dans le groupe constitué par les bactéries issues du genre *Lactococcus, Lactobacillus, Leuconotoc, Bifibobacterium, Pediococcus,* et/ou *Streptococcus.*

8. Méthode selon les revendications 4 à 7, dans laquelle l'étape b) est réalisée par centrifugation et/ou filtration tel que par ultrafiltration.

9. Méthode selon l'une quelconque des revendications 4 à 8, dans laquelle l'étape b) de concentration est réalisée par centrifugation.

10. Méthode selon les revendications 4 à 9, dans laquelle les étapes a) et b) sont effectuées une fois que la fermentation de la suspension contenant des microorganismes est terminée et/ou préalablement à un transfert quelconque dans d'autres récipients.

11. Méthode selon l'une quelconque des revendications 4 à 10, comprenant en outre les étapes suivantes :
c) éventuellement l'addition d'un agent cryoprotecteur au concentré contenant des microorganismes obtenu après les étapes a) et b) ;
d) la conservation ledit concentré contenant des microorganismes par refroidissement, congélation, séchage et/ou lyophilisation.

12. Méthode selon l'une quelconque des revendications 4 à 11, où ladite méthode comprend au moins les étapes suivantes :
- l'obtention d'une suspension contenant des microorganismes par un procédé de fermentation en mode discontinu ;
- l'addition d'un monosaccharide et/ou d'un disaccharide et/ou d'un alcool de sucre à ladite suspension contenant des microorganismes (étape a) ;
- la concentration de ladite suspension contenant des microorganismes, préférablement par centrifugation (étape b) ;
- la récupération dudit concentré contenant des microorganismes ou son transfert dans un autre récipient ;
- éventuellement, sa soumission à une étape de conservation telle que le refroidissement, la congélation, le séchage et/ou la lyophilisation.
